# EUROPEAN PATENT APPLICATION

(11) **EP 4 144 846 A1**
(43) Date of publication of application: **08.03.2023**
(21) Application number: 21194944.1
(22) Date of filing: 04.09.2021
(51) Int. Cl.: C12N 15/74

(54) **CARBON DIOXIDE-CONSUMING EXPRESSION SYSTEM**

(71) Applicant: 350 PPM Biotech GmbH, 20148 Hamburg (DE)
(72) Inventor: The designation of the inventor has not yet been filed
(74) Representative: Uexküll & Stolberg

(57) **Abstract**

The present invention generally relates to the field of heterologous protein production in host cells. In particular, the invention relates to a chemolithoautotrophic bacterium which has been genetically modified to produce one or more heterologous proteins. The invention also relates to a method for heterologous protein expression which makes use of the genetically modified chemolithoautotrophic bacterium of the invention. The invention further relates to the use of the genetically modified chemolithoautotrophic bacterium of the invention for heterologous protein expression. Finally, the invention provides a kit which comprises the genetically modified chemolithoautotrophic bacterium of the invention.

## Description

The present invention generally relates to the field of heterologous protein production in host cells. In particular, the invention relates to a chemolithoautotrophic bacterium which has been genetically modified to produce one or more heterologous proteins. The invention also relates to a method for heterologous protein expression which makes use of the genetically modified chemolithoautotrophic bacterium of the invention. The invention further relates to the use of the genetically modified chemolithoautotrophic bacterium of the invention for heterologous protein expression. Finally, the invention provides a kit which comprises the genetically modified chemolithoautotrophic bacterium of the invention.

### BACKGROUND OF THE INVENTION

It is widely recognized that human-induced carbon dioxide (CO₂) emission is the main cause of global warming. The German Federal Environment Agency estimated that industrial processes in Germany alone produce around 45 million tons of CO₂ emissions annually. To prevent the worst effects of global warming, international treaties were negotiated and adopted that sought to limit future emissions of greenhouse gases that contribute to global warming. Most significantly, the long-term temperature goal of the Paris climate agreement is to keep the rise in mean global temperature below 2 °C, and preferably below 1.5 °C above preindustrial levels.

In the Intergovernmental Panel on Climate Change (IPCC) climate report of 2021, it is assumed that it will not be possible to reach the 1.5°C temperature goal without so-called "negative emissions", i.e. the active removal of CO₂ from the atmosphere. One simple way of removing CO₂ from the atmosphere resides in afforestation. According to studies, 3.6 billion tons of CO₂ per year could be bound during the growth phase, which corresponds to about 10 percent of the present CO₂ emissions. However, afforestation requires very large areas, which means that areas presently used for agriculture would have to be converted, thereby interfering with the ability to produce food for a growing world population. Storing CO₂ deep underground is another well-known possibility of proving negative emissions. The CO₂ is first extracted from the ambient air by means of chemical processes and subsequently stored in underground geological formation, e.g. at the sea floor. This process is referred to as carbon capture and storage (CCS). Some CCS plants are already operating in Europe. While the CCS technology has great potential, it is presently extremely cost-intensive. The cost for removing a ton of CO₂ by CCS amounts to approximately 500-550 Euros. At present, the high costs are a barrier for using CCS in large scale.

Another idea that was discussed at a scientific level is the fertilization of the oceans with iron compounds. The rationale is to increase the nutrient content in the oceans such that plankton growth can be significantly increased which in turn leads to increased CO₂ binding. However, this idea is considered to involve a serious intervention into the ocean ecosystem which may be associated with severe and unpredictable side effects.

Another potential approach for providing negative emissions is the deployment of biotechnological processes that involve the consumption of CO₂. For example, it is known that certain microorganisms are able to convert CO₂ into organic carbon compounds. For example, it has been reported that autotrophic methanogenic archaea can consume CO₂ by converting it into methane. However, methanogenic archaea appear not to be suited for making products other than methane, another compound which contributes to global warming. Another group of microorganisms that efficiently utilize CO₂ are photoautotrophic bacteria, and in particular cyanobacteria (Kachel & Mack, 2020). However, these organisms grow relatively slowly which is mainly due to the technically difficult input of light energy.

Yet another interesting approach for CO₂ utilization is already in the pilot stage. The 'Rheticus' project jointly carried out by Siemens and Evonik aims at the biotechnological production of butanol and hexanol by chemolithotrophic organisms (Hoff et al., 2021). The technology uses electricity from renewable energy for the conversion of CO₂ into CO and H₂. CO₂, CO, and H₂ are fed into a three-stage fermentation process designed by Evonik using *Clostridium autoethanogenum* and *Clostridium kluyveri* finally generating a 1:1 mixture of butanol and hexanol. This mixture can either be separated by distillation or be employed as a fuel mixture. It is estimated that within five years, an industrial plant using this technology can produce 10.000 tons of hexanol and butanol per year using 25,000 tons of CO₂ as starting material.

In light of the high amounts of CO₂ that are emitted in the industrial countries, there is a high need for additional biotechnological processes that involve the consumption of CO₂. The biotechnological processes should preferably be feasible on an industrial scale so that as much CO₂ as possible is consumed. The process should be robust and easy to establish. In addition, the process should result in the production of a valuable product that can be used, e.g. in medicine, or as a food or feed.

The present invention addresses this need and provides additional advantages as well. The invention provides an expression host which can serve as a platform for establishing sustainable, CO₂-consuming biotechnological production processes. Importantly, the expression host can be genetically modified to produce one or more heterologous proteins, in particular human proteins. The expression host is a chemolithoautotrophic bacterium which is able to grow in the presence of CO₂ as a carbon source and hydrogen (H₂) as an energy source. Since the final acceptor for the reducing equivalents generated by the respiratory chain can be oxygen (O₂), the expression host of the present invention is classified as an aerobic organism.

### DESCRIPTION OF THE INVENTION

Thus, in a first aspect, the present invention provides a chemolithoautotrophic bacterium of the genus *Hydrogenovibrio* which has been genetically modified to produce one or more heterologous proteins. Preferably, the genetically modified chemolithoautotrophic bacterium belongs to the species *Hydrogenovibrio marinus.* More preferably, the genetically modified bacterium is derived from *H. marinus* MH-110, deposited at the Japanese Cell Collection (JCM) under deposition number JCM 7688. Bacterial microorganisms of the genus *Hydrogenovibrio* have high CO₂ binding capacities which render them suitable for use in a method of the present invention. For example, wild-type *H. marinus* contains three different ribulose 1,5-bisphosphate carboxylase/oxygenases (RubisCOs) which allow the organism to adapt to different CO₂ levels in the environment (Toyoda et al., 2005; Toyoda et al., 2018). While known aerobic hydrogen-oxidizing bacteria are sensitive to high oxygen levels and grow comparably slowly, *H. marinus* is able to grow rather quickly, up to a pO₂ of 40%. Optimum growth was observed at a pO₂ between 5 and 10%. The complete genome of *H. marinus* has been sequenced (Arai et al., 2019; Jo et al., 2014).

*H. marinus* MH-110 was isolated from a seawater sample collected at the coast of Shonan in Japan. At the time of isolation, *H. marinus* MH-110 was the first known mesophilic, obligate chemolithoautotrophic and aerobic hydrogen-oxidizing bacterium. The optimal observed growth temperature is 37 °C. At 5 °C or 45 °C no growth is observed. As a marine bacterium, *H. marinus* grows optimally in an aqueous growth medium at about 0.5-0.6 M NaCl with a pH preference of around 6.5. In principle, seawater of a suitable salinity can be used to prepare growth media for potential biotechnological production processes employing *H. marinus* which has the advantage that the risk for microbial contamination is further reduced. Notably, not many contaminating microorganisms are able to grow under chemolithoautotrophic conditions. Moreover, the use of seawater may be advantageous as it can be used in regions where freshwater is scarce.

The genetically modified bacteria of the invention are chemolithoautotrophic which means that they obtain the energy required for their growth from the oxidation of inorganic compounds such as H₂. At the same time, they are autotrophic which means that they are able to produce complex organic compounds using carbon from simple substances. In the case of bacteria from the genus *Hydrogenovibrio*, H₂ is used as an energy source and CO₂ as a carbon source. The particular advantage of *Hydrogenovibrio* is that it binds high amounts of CO₂ which means that using the organism in an industrial scale provides for the possibility of removing CO₂ from the ambient air, thereby rendering the process climate-friendly.

The present invention describes means and methods that can be used to generate genetically modified *Hydrogenovibrio* strains which produce one or more heterologous proteins. As used herein, a heterologous protein is a protein which is not encoded by a gene of the non-modified organism, i.e. the naturally occurring *Hydrogenovibrio* strain, and therefore not produced by the non-modified organism. The overproduction and extraction of a heterologous protein in cells of the genus *Hydrogenovibrio* has not been described so far. Hence, the present invention for the first time describes the generation of so-called production strains that can be used in large-scale biotechnological production processes. These strains allow the fully sustainable production of heterologous proteins from CO₂ and hydrogen gas. Preferably, the chemolithoautotrophic bacterium of the present invention has been genetically modified to produce a mammalian, more preferably a human protein. The protein preferably is a therapeutically active protein, i.e. a protein that can be used in human or veterinary medicine.

The type of therapeutically active protein is not particularly limited and can include cytokines, hormones, growth factors, and the like. In one embodiment, the host cells and methods of the invention are useful for the recombinant expression of an interferon protein. The type of interferon is not particularly limited. Generally, the interferon proteins selected for expression in the host cells of the invention can be of human or non-human origin. The interferon protein can belong to any interferon subclass that is known in the art. In particular, the interferon protein to be expressed in the host cell of the invention can be an alpha-interferon (IFN-α), a beta-interferon (IFN-β) or a gamma-interferon (IFN-γ). In a particularly preferred embodiment, the interferon protein to be expressed is human IFN-α, IFN-β or IFN-γ or a biologically active fragment thereof.

In a preferred embodiment, the mammalian protein is human somatotropin (hGH) or a biologically active fragment thereof. As shown herein, it was possible to create a *H. marinus* strain that produces human somatotropin, a protein that is also referred to in the literature as human growth hormone (hGH). Somatotropin is a protein that is produced by the pituitary gland in the brain and stimulates the liver to produce somatomedins, i.e. hormones that are very similar in structure to insulin and stimulate the growth of bones. Children and adolescents normally produce large quantities of somatotropin. hGH is normally produced as a precursor of 217 amino acids. The major isoform of the hGH is a protein of 191 amino acids and a molecular weight of 22,124 daltons. The structure includes four helices necessary for functional interaction with its cognate growth hormone receptor. Recombinant hGH (rhGH) was shown to be biologically active when produced in prokaryotic systems e.g. in *Escherichia coli* (Olson et al., 1981). This indicates that posttranslational modifications which occur in e.g. mammalian cells are not necessary to generate an active pharmaceutical ingredient. rhGH produced in *E. coli* was approved by the FDA in 1985. As of 2005, different rhGH products were available in the market from manufacturers like Genentech, Eli Lilly, Pfizer, Merck Serono, and Sandoz.

In a preferred embodiment the chemolithoautotrophic bacterium of the present invention has been modified to produce an hGH protein that comprises or consists of
(a) the amino acid sequence of SEQ ID NO: 1, or
(b) an amino acid sequence having at least 80% sequence identity to the amino acid sequences of SEQ ID NO: 1.

Preferably, the hGH protein produced by the chemolithoautotrophic bacterium of the present invention is the protein depicted in SEQ ID NO:1. Alternatively, the hGH protein produced by the chemolithoautotrophic bacterium can be a variant of the protein depicted in SEQ ID NO:1 shares a high degree of sequence identity of at least 80%. More preferably, the sequence identity is 91%, 92%, 93%, 94%, 95%, 96%, 97%, 98%, or 99%. Preferably, the sequence identity is determined over a length of at least 50 amino acids, more preferably at least 75 amino acids, at least 100 amino acids, at least 125 amino acids, at least 150 amino acids, at least 175 amino acids, or at least 200 amino acids. More preferably, the sequence identity is determined over a full length of the precursor protein, i.e. 217 amino acids.

In one embodiment, the hGH variant shares a sequence identity with the amino acid sequence of SEQ ID NO:1 of at least 95% over a length of at least 50 amino acids, preferably at least 75 amino acids, more preferably at least 100 amino acids, even more preferably at least 125 amino acids, even more preferably at least 150 amino acids, even more preferably at least 175 amino acids, and even more even more preferably at least 200 amino acids. It is particularly preferred that the sequence identity is at least 95% over the full length of the protein. In one embodiment, the hGH variant shares a sequence identity with the amino acid sequence of SEQ ID NO:1 of at least 96% over a length of at least 50 amino acids, preferably at least 75 amino acids, more preferably at least 100 amino acids, even more preferably at least 125 amino acids, even more preferably at least 150 amino acids, even more preferably at least 175 amino acids, and even more preferably at least 200 amino acids. It is particularly preferred that the sequence identity is at least 96% over the full length of the protein. In one embodiment, the hGH variant shares a sequence identity with the amino acid sequence of SEQ ID NO:1 of at least 97% over a length of at least 50 amino acids, preferably at least 75 amino acids, more preferably at least 100 amino acids, even more preferably at least 125 amino acids, even more preferably at least 150 amino acids, even more preferably at least 175 amino acids, and even more preferably at least 200 amino acids. It is particularly preferred that the sequence identity is at least 97% over the full length of the protein. In one embodiment, the hGH variant shares a sequence identity with the amino acid sequence of SEQ ID NO:1 of at least 98% over a length of at least 50 amino acids, preferably at least 75 amino acids, more preferably at least 100 amino acids, even more preferably at least 125 amino acids, even more preferably at least 150 amino acids, even more preferably at least 175 amino acids, and even more preferably at least 200 amino acids. It is particularly preferred that the sequence identity is at least 98% over the full length of the protein. In one embodiment, the hGH variant shares a sequence identity with the amino acid sequence of SEQ ID NO:1 of at least 99% over a length of at least 50 amino acids, preferably at least 75 amino acids, more preferably at least 100 amino acids, even more preferably at least 125 amino acids, even more preferably at least 150 amino acids, even more preferably at least 175 amino acids, and even more preferably at least 200 amino acids. It is particularly preferred that the sequence identity is at least 99% over the full length of the protein.

In order to determine the sequence identity between two amino acid sequences, these sequences are usually aligned for optimal comparison. For example, gaps can be introduced in the sequence of a first amino acid sequence for optimal alignment with a second amino acid sequence. The amino acids at corresponding positions are then compared. If identical amino acids occur in corresponding positions in the first and second amino acid sequence, the sequences are identical at that position. A percentage sequence identity between two amino acid sequences means that, when aligned, the recited percentage of amino acids are identical in comparing both sequences. A percentage sequence identity can be determined by using software programs that are widely known in the art, for example the ALIGN program (version 2.0), which is part of the GCG sequence alignment software package. When utilizing the ALIGN program for comparing amino acid sequences, a PAM120 weight residue table, a gap length penalty of 12, and a gap penalty of 4 can be used.

Variants of the protein depicted in SEQ ID NO:1 also include proteins which differ from the original hGH by one or more (e.g. 2, 3, 4, 5, 10, or 15) additional amino acids. These additional amino acids may be present within the amino acid sequence of the original hGH protein (i.e. as an insertion), or they may be added to one or both termini of the protein. Basically, insertions can take place at any position provided that the addition of amino acids does not impair the capability of the protein to exert at least part of the biological activity that is exerted by the naturally occurring hGH protein. Similarly, variants also comprise those proteins in which, compared to the original protein, one or more amino acids are lacking. Such deletions may affect any amino acid position provided that they do not impair the capability of the variant to exert at least part of the biological activity that is exerted by the naturally occurring hGH protein.

Variants of the hGH protein also encompass proteins with structural modifications relative to the naturally occurring protein, such as modified amino acids. According to the invention, modified amino acids may comprise phosphorylation, glycosylation, acetylation, acylation, branching, ADP ribosylation, crosslinking, disulfide bridge formation, formylation, hydroxylation, carboxylation, methylation, demethylation, amidation, cyclization and/or covalent or non-covalent bonding to phosphatidylinositol, flavine derivatives, lipoteichonic acids, fatty acids or lipids. Such modifications have been extensively described in the literature, e.g., in Proteins: Structure and Molecular Properties, T. Creighton, 2nd edition, W. H. Freeman and Company, New York (1993).

In a preferred embodiment, the chemolithoautotrophic bacterium of the invention comprises a plasmid. As used herein, a plasmid refers to an extrachromosomal circular DNA capable of autonomous replication in a cell. The plasmid can be an integrating or non-integrating plasmid. However, the use of a plasmid which does not integrate into the genome of the host cell is preferred. In a preferred embodiment the plasmid is an expression vector which means that it carries a nucleotide sequence that encodes the heterologous protein to be produced by the host cell. Expression vectors are typically DNA constructs that are optimized for protein expression and comprise an origin of replication that allows the vector to replicate independently of the host cell. Expression vectors also include regulatory control elements that are functionally linked to the nucleotide sequences to be expressed and control their transcription and/or translation. Suitable control elements include constitutive or controllable prokaryotic promoters, a transcription termination sequence and a ribosome binding site. Expression vectors may also include one or more antibiotic resistance genes which allow transformed cells to be selected by use of media that contain antibiotics.

The plasmid or expression vector comprised by the chemolithoautotrophic bacterium of the invention preferably comprises a synthetic promoter, more preferably a promoter selected from the group of J23119 (SEQ ID NO:8), J23111 (SEQ ID NO:9) and J23114 (SEQ ID NO:10). The plasmid or expression vector comprised by the chemolithoautotrophic bacterium of the invention preferably also comprises a synthetic ribosomal binding site, more preferably a ribosomal binding site selected from the group of BBa_B0034 (SEQ ID NO:11), BBa_B0064 (SEQ ID NO:12),BBa B0031 (SEQ ID NO:13), and *ribDG* (SEQ ID NO:14). A plasmid or expression vector comprising the above-recited components preferably is a non-integrative plasmid.

The expression vector comprised by the chemolithoautotrophic bacterium of the invention further comprises a gene that codes for the heterologous protein. The size of the heterologous protein encoded by the gene is not particularly limited and preferably ranges from 50 to 1200 amino acids, more preferably from 75 to 1000 amino acids, from 100 to 900 amino acids,from 150 to 800 amino acids, from 175 to 750 amino acids, or from 200 to 600 amino acids. The heterologous protein can encode a therapeutically or enzymatically active or a fragment of such a protein, provide that the fragment is still therapeutically or enzymatically active.

The plasmid or expression vector can be cloned in accordance with routine methods known in the art which are described, e.g. in Maniatis et al. 1982, Molecular Cloning, A laboratory Manual, Cold Spring Harbor Laboratory. The plasmid or expression vector is preferably introduced into the chemolithoautotrophic bacterium of the invention by parental mating with a suitable donor strain. For this purpose, the plasmid or expression vector is first introduced into a *E. coli* stain, such as *E. coli* WM3064 by routine methods, such as electroporation or calcium phosphate- or rubidium chloride-mediated transformation. In a subsequent step, the plasmid or expression vector is transferred from the donor strain WM3064 to the *Hydrogenovibrio* strain by spreading and incubating aliquots from cultures of both strains on a nitrocellulose filter, as described below in Example 3.

In a second aspect, the invention provides a method for the production of one or more heterologous proteins in a host cell, said method comprising the following steps
(a) providing a chemolithoautotrophic bacterium as described hereinabove;
(b) culturing the host cell under conditions that allow the production of the heterologous protein;
(c) obtaining the heterologous protein.

The method of the invention is directed to the production of one or more heterologous proteins in a host cell. Preferably, the method seeks to produce a single heterologous protein. The method makes use of genetically modified chemolithoautotrophic *Hydrogenovibrio* bacterium described above. Preferably, the genetically modified chemolithoautotrophic bacterium belongs to the species *Hydrogenovibrio marinus.* More preferably, the genetically modified bacterium is derived from *H. marinus* MH-110, deposited at the Japanese Cell Collection (JCM) under deposition number JCM 7688.

As used herein, a bacterium is considered to belong to the species *H. marinus* if the 16S rRNA comprises the
(a) the nucleotide sequence of SEQ ID NO:6, or
(b) a nucleotide sequence having at least 99% or more sequence identity to the nucleotide sequences of SEQ ID NO:6.

Preferably, the nucleotide sequence has at least 99% or more sequence identity to the nucleotide sequences of SEQ ID NO:6 over a length of 400 nucleotides, 500 nucleotides, 600 nucleotides, 700 nucleotides, 800 nucleotides, 900 nucleotides, or 1000 nucleotides of the sequence of SEQ ID NO:6. More preferably, the nucleotide sequence has at least 99% or more sequence identity to the nucleotide sequences of SEQ ID NO:6 over the full length of the 1402 nucleotides.More preferably, the nucleotide sequence has at least 99.5% or more sequence identity to the nucleotide sequences of SEQ ID NO:6 over a length of 400 nucleotides, 500 nucleotides, 600 nucleotides, 700 nucleotides, 800 nucleotides, 900 nucleotides, or 1000 nucleotides of the sequence of SEQ ID NO:6. Even more preferably, the nucleotide sequence has at least 99.5% or more sequence identity to the nucleotide sequences of SEQ ID NO:6 over the full length of the 1402 nucleotides. More preferably, the nucleotide sequence has at least 99.8% or more sequence identity to the nucleotide sequences of SEQ ID NO:6 over a length of 400 nucleotides, 500 nucleotides, 600 nucleotides, 700 nucleotides, 800 nucleotides, 900 nucleotides, or 1000 nucleotides of the sequence of SEQ ID NO:6. Even more preferably, the nucleotide sequence has at least 99.8% or more sequence identity to the nucleotide sequences of SEQ ID NO:6 over the full length of the 1402 nucleotides.

The *Hydrogenovibrio* bacterium of the invention is cultured under conditions that allow the production of the heterologous protein. Such conditions include conditions of temperature, pH and salt that allow for the growth and propagation of the cells. For *Hydrogenovibrio marinus,* an appropriate growth temperature will be in the range of 30-39 °C, whereas a growth temperature of 36-37 °C is particularly preferred. The media used for cell culturing will include a certain amount of salt. Preferably, the media used for culturing contain 0.2-1.0 M NaCl, and more preferably 0.5-0.6 M NaCl. A pH in the range of 6.0-8.0 is preferred, more preferably 7.0-8.0, such as 6.5.

Cell culturing is performed in a gaseous atmosphere containing H₂, CO₂ and oxygen (O₂). A wide variety of different ratios of the three gases can be used for generating the atmosphere used for culturing. Preferably, atmosphere compositions are avoided that contain 4% or more O₂ and, at the same time, 4% or more H₂, since such atmosphere compositions are explosive which complicates the culturing of the cells. It is therefore preferred that either O₂ or H₂ or both are used in an amount of less than 4% when producing the atmosphere compositions for cell culturing.

In one embodiment, H₂ is present in the atmosphere composition for cell culturing in an amount of 80-89%, more preferably 85-89%, and even more preferably 87-89%. In such embodiment, the CO₂ is present in the atmosphere composition in an amount of 2-10%, more preferably 5-10%, and even more preferably 8-10 %. In such embodiment, the O₂ is present in the atmosphere composition in an amount of 0.5-3%, more preferably 1-3%, and even more preferably 2-3 %. The atmosphere composition may also include N₂ in an amount of up to 2%. Preferred atmosphere compositions include H₂:CO₂:O₂:N₂ = 89:10:1:0, 88:10:2:0, and 87:10:3:0. Alternative preferred atmosphere compositions include H₂:CO₂:O₂:N₂ = 82:15:3:0, 83:15:2:0, and 84:15:1:0.

In another embodiment, H₂ is present in the atmosphere composition for cell culturing in an amount of 70-79%, more preferably 75-79%, and even more preferably 77-79%. In such embodiment, the CO₂ is present in the atmosphere composition in an amount of 2-10%, more preferably 5-10%, and even more preferably 8-10 %. In such embodiment, the O₂ is present in the atmosphere composition in an amount of 2-10%, more preferably 5-10%, and even more preferably 8-10%. The atmosphere composition may also include N₂ in an amount of up to 5%. Preferred atmosphere compositions include H₂:CO₂:O₂:N₂ = 77:10:10:3, 78:10:10:2, and 79:10:10:1.

In yet another embodiment, H₂ is present in the atmosphere composition for cell culturing in an amount of 0.5-3%, more preferably 1-3%, and even more preferably 2-3%. In such embodiment, the CO₂ is present in the atmosphere composition in an amount of 2-10%, more preferably 5-10%, and even more preferably 8-10 %. In such embodiment, the O₂ is present in the atmosphere composition in an amount of 2-10%, more preferably 5-10%, and even more preferably 8-10%. The atmosphere composition may also include N₂ in an amount of up to 80%. Preferred atmosphere compositions include H₂:CO₂:O₂:N₂ = 3:9:8:80, 3:8:9:80, 3:9:9:79, and 2:10:10:78.

Conveniently, it is also possible to culture the *Hydrogenovibrio* strains of the invention in a gaseous atmosphere that comprises ambient air (which includes about 78% N₂, about 21% O₂ and some other gases) that has been supplemented with 8-10% CO₂ and 1-3% H₂. Preferably, the gaseous atmosphere comprises ambient air that has been supplemented with 10% CO₂ and 3% H₂ or ambient air that has been supplemented with 10% CO₂ and 2% H₂.

The *Hydrogenovibrio* strain of the invention is cultured until a suitable OD has been reached, preferably an OD₅₄₀ of 0.3-1.8, more preferably an OD₅₄₀ of 0.5-1.6, and even preferably an OD₅₄₀ of 0.7-1.0. Once a sufficiently high OD has been reached, the culturing is stopped and the protein is harvested.

In step (c) of the method, the heterologous protein is obtained from the host cell. Typically, the protein is isolated from the cytosol of the host cells. Preferably, step (c) of the method comprises the disruption of the cells, e.g., by a French press or bead mill, by repeating cycles of freezing and thawing, or by sonication. Cellular debris can be easily removed from the protein fraction by centrifugation. The heterologous protein can then be purified from other protein and non-protein components by standard chromatography methods, including, for example, size exclusion chromatography and/or ion exchange chromatography. Other methods which are typically applied in protein purification, such as ammonium sulfate precipitation and filtration and/or dialysis may also be used. The heterologous protein expressed by the host cell of the invention may also be purified by affinity purification using, e.g., monoclonal or polyclonal antibodies.

In a preferred embodiment, step (c) of the method of the invention comprises the purification of the heterologous protein. To simplify purification, the heterologous protein produced according to the method of the invention may be expressed as a fusion protein which comprises an affinity tag that facilitates binding of the fusion protein via a compound exhibiting binding affinity to the tag. For example, the affinity tag may be a poly-histidine tag comprising 6-12 histidine residues which specifically interacts with a nickel ion chelate matrix. Alternatively, the tag may be glutathione-S-transferase allowing the purification on a glutathione matrix. Further affinity tags are well-known in the art. Non-limiting examples for pairs of affinity tag and affinity ligand include maltose-binding-protein (MBP) and maltose; avidin and biotin; Streptag and streptavidin; myc epitope and antibody. Where the affinity tag is a peptide or polypeptide, such tag may conveniently be expressed together with the heterologous protein as a single expression product. Where the affinity tag is not of protein origin, it may be attached by chemical coupling reactions to the heterologous protein. For example, biotin may be chemically coupled to the heterologous protein.

In the event that the genetically modified *Hydrogenovibrio* bacterium of the invention is used for the production of a therapeutically or enzymatically active protein, it is preferred that at least 50% of the heterologous protein produced according to the method of the invention is therapeutically or enzymatically active protein. More preferably, at least 60%, at least 70%, at least 80%, at least 90%, or at least 95% of the heterologous protein obtained from the method of the invention is therapeutically or enzymatically active.

In a third aspect, the invention relates to the use of the chemolithoautotrophic bacterium described above as a host cell for the production of a heterologous protein.

In a fourth aspect, the invention relates to a kit for carrying out a method. In particular, the invention relates to a kit for the production of a heterologous protein comprising the chemolithoautotrophic bacterium described above. The kit comprises the chemolithoautotrophic bacterium described hereinabove, i.e. a chemolithoautotrophic *Hydrogenovibrio* bacterium which has been genetically modified to produce one or more heterologous proteins. The kit further comprises media and reagents that are suitable for culturing the *Hydrogenovibrio* bacterium and initiating gene expression.

### BRIEF DESCRIPTION OF THE FIGURES

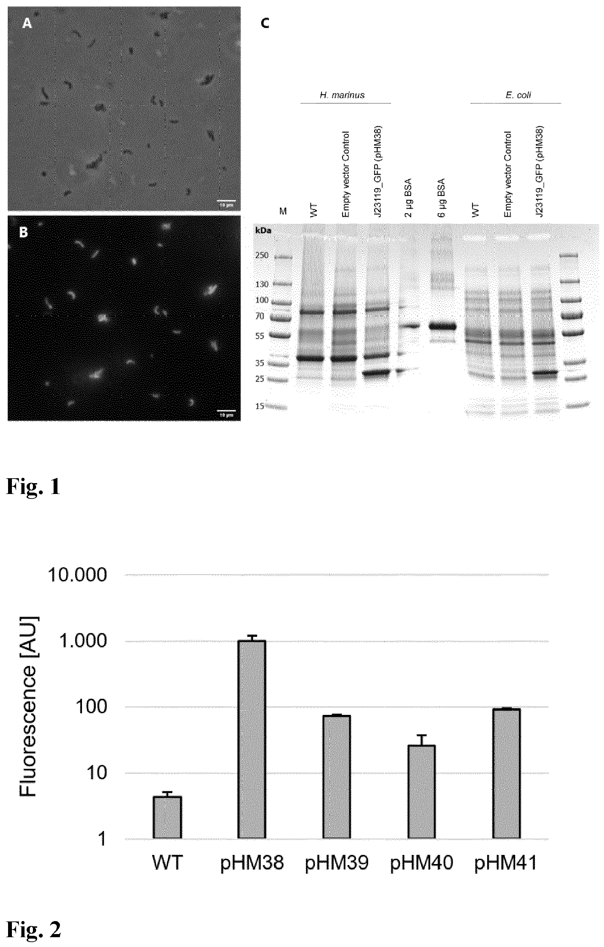

**Figure 1** shows the production of sf-GFP in *Hydrogenovibrio marinus.* (A) Phase contrast microscopy confirms that cells of a recombinant *H. marinus* strain containing the expression plasmid pHM38 show the same characteristic shape as the wild-type. (B). Fluorescence of sf-GFP was excited at a wavelength of 485 nm and emission was captured at 525 nm. (C) SDS-PAGE analysis followed by Coomassie Brilliant Blue R-250 staining of the soluble fraction of recombinant *H. marinus* cell lysates reveals a strong protein band at about 26 kDa in case of the *H. marinus* strain expressing J23119_GFP (pHM38) corresponding to the expected size of sf-GFP. Cell-free extracts of recombinant *E. coli* WM3064 carrying either the empty expression vector or pHM38, respectively, contain a similar band with a similar strength. Cellular lysates containing 16 µg of total protein were loaded onto the gel. A bovine serum albumin (BSA) standard was used as a control and in order to estimate the amount of sf-GFP protein in the cellular lysate of the recombinant *H. marinus* strains (M: PageRuler pre-stained protein standard).
**Figure 2** shows the result of measuring sf-GFP fluorescence in recombinant *H. marinus* strains expressing the gene encoding sf-GFP under the control of different promoter-RBS variants. Error bars indicate one standard deviation from average of n=4 technical replicates.

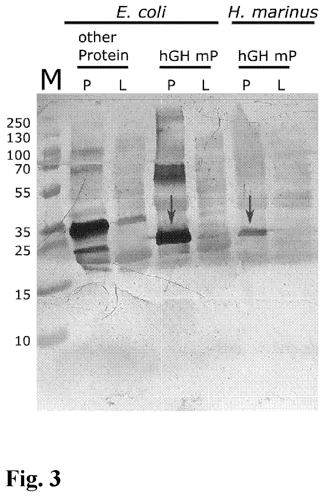

**Figure 3** shows the detection of rhGH by Western Blot in cell-free extracts of *E. coli* and *H. marinus.* Protein extracts from the insoluble (P) and soluble (L) fractions of the cultures producing either His-tagged rhGH or another His-tagged protein from a moderate promoter (mP) were probed via a HRP-conjugated 6xHis antibody and detected with 3,3'-diaminobenzidine (DAB) substrate solution.

### EXAMPLES

The present invention is further described in more detail by the following examples which are only provided for illustrating the invention and which are not to be construed as limiting the scope of the invention. The following material and methods were used in the Examples.

### Example 1: Culturing of H. marinus

*H. marinus* strain MH-110 is obtainable from the Japanese Cell Collection (JCM) under deposition number JCM 7688. The strain was cultured in a Basal Medium (Nishihara et al., 1991), with a final composition of 2 g/L K₂HPO₄, 1 g/L KH₂PO₄, 5 g/L (NH₄)₂SO₄, 29.3 g/L NaCl, 0.2 g/L MgSO₄ • 7H₂O, 10 mg/L CaCl₂, 10 mg/L FeSO₄ • 7H₂O, 0.6 mg/L NiSO₄ • 7H₂O and 2 mL/L trace metal solution containing 1.68 mg/L Na₂MoO₄ • 2H₂O, 7 mg/L ZnSO₄ • 7H₂O, 0.5 mg/L CuSO₄ • 5H₂O, 1 mg/mL H₃BO₃, 0.66 mg/L MnSO₄ • 5H₂O, 1 mg/L CoCl₂ • 6H₂O. *H. marinus* strain MH-110 was cultivated as 50 mL cultures in 300 mL Erlenmeyer baffled flasks, or in small culture volume (1 mL) in 24-well cell culture plates using a gaseous atmosphere containing H₂, CO₂ and O₂. To prevent cells from forming aggregates, a glass bead of ca. 3 mm diameter was added to each 1 mL culture. The Erlenmeyer flask, or the 24-well plate, respectively, was then placed into a 2 L anaerobic jar (anaerobic jar "small", Cat.-No. 3.380.202, Schuett-Biotec GmbH, Germany) which was filled with a gaseous atmosphere of H₂, CO₂ and O₂ at different ratios (H₂:CO₂:O₂:N₂ = 87:10:3:0; H₂:CO₂:O₂:N₂ = 88:10:2:0 and H₂:CO₂:O₂:N₂ = 3:10:10:77). The jar was placed in an incubator set at 37°C, 150 rpm. The atmosphere was refreshed every 24 hours. The optical density of the cultures was measured using a photometer set to a wavelength of 540 nm (OD₅₄₀).

Results: Growth of *H. marinus* strain MH-110 was observed in all of the tested atmospheres. It was found however that the atmosphere H₂:CO₂:O₂:N₂ = 88:10:2:0 generated more biomass in a shorter time compared to cultures cultivated in a low-hydrogen atmosphere of H₂:CO₂:O₂:N₂ = 3:10:10:77.

### Example 2: Species validation of H. marinus by colony PCR and 16S rDNA sequencing.

*H. marinus* MH-110 was grown in a medium as set out above in Example 1 which was solidified by the addition of 1.5% agar-agar (powder, BioScience, BioScience Grade, Carl Roth). For the culturing on plates, the atmosphere was set to H₂:CO₂:O₂:N₂ = 88:10:2:0. *H. marinus* colonies from agar plates were picked and subjected to colony PCR using primers which amplify indicative parts of genes encoding bacterial 16S rRNAs.

For that purpose, 32.5 µl double distilled H₂O, 10 µl 5x Phusion Hot Start II DNA Polymerase buffer, dNTP Mix (10 mM each, Thermo Scientific), 2.5 µl of each primer (10 µM stock), 0.5 µl Phusion Hot Start II DNA Polymerase (Thermo Scientific) were mixed in a 200 µl single use plastic PCR tube. Cell material obtained from an agar plate was picked with a sterile pipette tip and transferred to the PCR tube. The PCR was then performed with the following settings: 1 min at 98°C followed by 35 cycles of 10 sec at 98 °C, 20 sec at 53 °C, 45 sec at 72 °C, followed by 6 min at 72 °C. The reaction subsequently was kept at 4 °C prior to further analysis. The following primers were used:
Forward primer (SEQ ID NO:4): 5'-AGAGTKTGATCMTGGCTCAG-3
Reverse primer (SEQ ID NO:5): 5'-GACGGGCGGTGTGTRCA-3'.

Primer mixtures with variable positions were used. The primers do not exactly match their corresponding target DNA which renders them useful for generating PCR products from a variety of bacterial species especially, including yet unknown species. The parts of the 16S rRNA genes which are PCR-amplified by using these primers are considered to be indicative for a specific bacterial species (K = G or T; M = A or C; R = A or G). The resulting PCR product was sequenced with the above primers, and the sequence of the PCR product was determined. The sequence of the PCR product was analyzed using the Nucleotide Basic Local Alignment Search Tool (BLASTN) and the nucleotide collection (nr/nt) provided by the National Center for Biotechnology Information (NCBI). The BLASTN algorithm identifies regions of local similarity between sequences.

Results: The amplified DNA fragment (1402 nucleotides) showed 100% percent sequence identity to the 16S rRNA gene of *H. marinus.* This was understood as evidence that the colony was a *H. marinus* colony. The nucleotide sequence of the 16S rRNA gene of *H. marinus* is depicted in SEQ ID NO:6.

### Example 3: Construction of a sf-GFP-producing strain of H. marinus

All expression plasmids used in this experiment contain the pAM5409 backbone (Bishé, et al., 2019). A GFP expression cassette was created, consisting of a gene encoding a strep-tagged superfolder (sf)-GFP (Pédelacq et al., 2006) driven by the synthetic promoter BBa_J23119, combined with the ribosomal binding site (RBS) BBa_B0034 (both derived from the iGEM Registry of Standard Biological Parts), and terminated by the terOOP terminator sequence amplified from the pUR_dualTag plasmid obtained from Prof. Wilde (University of Freiburg, Germany). The nucleotide sequence of the sf-GFP-expression cassette is set out herein as SEQ ID NO:7. The sequence of the sf-GFP-expression cassette was cloned into the pAM5409 plasmid backbone resulting in the plasmid pHM38.

Recombinant *H. marinus* strains were created via parental mating with an *E. coli* WM3064 donor strain carrying the pAM5409-based expression plasmids. For this purpose, a *H. marinus* MH-110 culture was inoculated from a cryostock in Basal Medium and cultured under standard autotrophic growth conditions to an OD₅₄₀ of 0.6-1.4 and, subsequently, concentrated to an OD₅₄₀ of 20-30. *E. coli* WM3064 donor strain carrying plasmid pHM38 was cultured overnight in LB medium supplemented with 0.3 mM diaminopimelic acid (DAP) and 50 µg/mL kanamycin. The culture was washed twice in LB medium + 0.3 mM DAP, cells were suspended in LB + 0.3 mM DAP and incubated for 15 min at 37°C and 180 rpm. The culture was then concentrated 10-fold. 100 µL of the concentrated *H. marinus* and *E. coli* cultures were mixed and spread on a 0.45 µm nitrocellulose filter (Carl Roth, Germany) placed on a Basal Medium 1% agar plate supplemented with 0.3 mM DAP and 5% LB. The plate was incubated under standard autotrophic growth conditions for 16 hours. The conjugation mixture was then washed from the filter using 500 µL of Basal Medium containing 15 µg/mL kanamycin. 100 µL of the suspension was used to inoculate 900 µL Basal Medium containing 15 µg/mL kanamycin and cultured in a 24-well cell culture plate under standard autotrophic growth conditions for approx. three days. The cultures were then diluted 1/10 in fresh Basal Medium containing 15 µg/mL kanamycin and incubated under standard autotrophic growth conditions for two days. Incubation was carried out in the medium and under conditions described in Example 1 for agar plates and 24-well plates. The gas atmosphere was set to H₂:CO₂:O₂:N₂ = 88:10:2:0.

The conjugation procedure was considered successful when
a) growth was observed in selection medium containing 15 µg/mL kanamycin and the liquid cultures reached an OD₅₄₀ between 0.5 to 1.4,
b) 16S rRNA sequencing of a cell sample confirmed the presence of *H. marinus,*
c) no growth on LB agar plates was observed (thereby excluding contamination with *E*. *coli* or other heterotrophic microorganisms).

The purity of the conjugated *H. marinus* strains was confirmed by phase contrast light microscopy (100x) and 16S-rRNA sequencing using primers set out above in SEQ ID NO:4 and SEQ ID NO:5. Additionally, the sample was streaked on a LB agar plate and incubated in the presence of air at 37 °C for several days to confirm that the culture was pure of viable *E. coli* cells.

The liquid cultures derived from the conjugation/selection procedure were analyzed by fluorescence microscopy. The samples were excited using light of a wavelength of 485 nm and the emitted fluorescence was captured at a wavelength of 535 nm. To quantify the sf-GFP fluorescence emitted by the recombinant strains, 100 µL of the cultures were analyzed in a black flat-transparent bottom 96-well plate (Greiner BioOne, Germany) using the GeniusPro^{®} plate reader (Tecan Group, Switzerland) set to 485 nm excitation and 535 nm emission wavelength.

Results: The results are depicted in Fig. 1A and B. It can be seen that only the *H. marinus* cells which contained the full reporter gene construct show strong fluorescence.

### Example 4: Analysis of the sf-GFP-expressing H. marinus strain by SDS-PAGE

The recombinant *H. marinus* strain carrying pHM38 (J23119_GFP) was analyzed by polyacrylamide gel electrophoresis in the presence of sodium dodecyl sulfate (SDS-PAGE). The strain was plated on a Basal Medium agar plate supplemented with 15 µg/mL kanamycin and incubated under standard autotrophic growth conditions for five days. The biofilm was collected and resuspended in 400 µL of 1x Buffer W (IBA Lifesciences GmbH, Germany). Approx. 200 µL silica beads (size 0.1-0.2 mm) were added and cells were disrupted via bead beating in a FastPrep device (MP Biomedicals Germany GmbH) using three cycles at 7.5 m/s for 45 sec with 5 min breaks on ice. The cell debris was separated from the soluble fraction via centrifugation at 14,000 rpm and 4 °C for 10 min, and the protein content of the soluble fraction was measured using the standard Bradford method. 16 µg of total protein were mixed with reducing 4x RotiLoad loading buffer (Carl Roth, Germany) and incubated at 95 °C for 15 minutes. Samples were loaded on a NuPAGE^{™} 4-20% polyacrylamide gel (Thermo Scientific Fisher). PageRuler^{™} Prestained Protein Ladder was used as protein size reference. Standard SDS-Running Buffer (final concentration: 0.25 M Tris-base, 1.924 M Glycine, 0.347 M SDS) was used. SDS-PAGE was performed at 100 V for 20 min followed by 200 V for ca. 30-60 min. The resulting gel was stained with PageBlue^{™} Protein Staining Solution (Thermo Scientific) overnight at room temperature. The gel was washed with de-ionized water until protein bands became distinguishable.

Results: The results are depicted in Fig. 1C. When compared to the wild-type control the recombinant *H. marinus* strain containing the full reporter gene construct produced an additional protein band corresponding to a signal in the SDS gel at around 26 kDa. The corresponding protein band was excised. Subsequently, the resulting peptides were subjected to analysis by mass spectrometry. The masses of the resulting peptides exactly matched the expected masses of an *in silico* digested sf-GFP. Thus, the results show that sf-GFP is produced by the recombinant *H. marinus* strain. Staining of the gel with Coomassie R 250 brilliant blue revealed that the recombinant protein (sf-GFP) was produced by the recombinant cells in comparably large amounts. The amount of sf-GFP in the soluble fraction of the recombinant *H. marinus* pHM38 cell lysate is comparable to what is present in an *E. coli* strain containing a similar construct. When comparing the total soluble protein amount of the bovine serum albumin (BSA) standard to the total soluble protein amount of sf-GFP detected in a cell-free extract of e.g. *H. marinus* pHM38, it is estimated that heterologous sf-GFP represents about 18-31% of the total protein in the soluble fraction of the recombinant *H. marinus* strain. This can be estimated upon SDS-PAGE and Coomassie Brilliant Blue R-250 staining.

### Example 5: Construction of sf-GFP-expressing strains using synthetic promoter variants

To examine whether the expression levels can be modulated, different synthetic promoters and ribosomal binding sites were combined. The promoter sequences J23119 (SEQ ID NO:8), J23111 (SEQ ID NO:9) and J23114 (SEQ ID NO: 10) were derived from the iGEM parts registry and combined with different ribosomal binding sites. The ribosomal binding site sequences named BBa_B0034 (SEQ ID NO:11), BBa_B0064 (SEQ ID NO:12) and BBa_B0031 (SEQ ID NO:13) were also derived from the iGEM parts registry. Moreover, a ribosomal binding site derived from the riboflavin biosynthetic gene *B. subtilis ribDG* was used (SEQ ID NO:14).

The GFP-expression plasmid (pHM38) described in Example 3 was used. To create variants containing different promoters and RBSs, pHM38 was digested using *Kpn*I/*Nhe*I*.* The oligonucleotides DNA_039 (SEQ ID NO:15)/DNA_040 (SEQ ID NO:16), containing the J23111 promoter and the BBa_B0064 RBS, were annealed and ligated into the digested backbone, to generate plasmid pHM39. The oligonucleotides DNA_041 (SEQ ID NO:17)/DNA_042 (SEQ ID NO:18), containing the J23114 promoter and the BBa_B0031 RBS, were annealed and ligated into the digested backbone, to generate plasmid pHM40. The oligonucleotides DNA_043 (SEQ ID NO:19)/DNA_044 (SEQ ID NO:30), containing the J23119 promoter and the RBS derived from the *B. subtilis ribDG* gene, were annealed and ligated into the digested backbone, to generate plasmid pHM41. The sequence of pHM38, pHM39, pHM40 and pHM41 are depicted as SEQ ID NO:22, 23, 24 and 25, respectively.

*H. marinus* wild-type strain was conjugated with the plasmids pHM38 - pHM41, respectively, using the conjugation method described above. *H. marinus* strains carrying the respective plasmids were inoculated to an OD₅₄₀ of 0.01 and cultured under standard autotrophic growth conditions for four days. sf-GFP fluorescence was measured using the GeniusPro^{™} plate reader.

Results: The results are depicted in Fig. 2 and in the below Table 1. The data show elevated sf-GFP levels for all recombinant *H. marinus* strains in comparison to the wild type. The data suggest that depending on the choice of the promoter-RBS variant, a low level of expression, a medium level of expression or a high level of expression of the heterologous gene occurred.

**Table 1: Fluorescence of sf-GFP-expressing H. marinus strains in comparison to the wild-type (WT)**

| **Strain** | **Expected promoter strength** | **Expected RBS strength** | **sf-GFP-fluorescence fold-change** | **Stdev** |
|---|---|---|---|---|
| WT | - | - | 1 | n.a. |
| pHM38 | Strong | Strong | 227 | 47.2 |
| pHM39 | Moderate | Moderate | 17 | 0.6 |
| pHM40 | Weak | Weak | 6 | 2.6 |
| pHM41 | Strong | Weak | 21 | 0.9 |

It is thus possible to achieve different production levels of a certain heterologous protein depending on the combination of the genetic elements.

### Example 6: Overexpression of human somatotropin (rhGH)

*H. marinus* MH-110 was also modified to produce recombinant human somatotropin (rhGH). The sequence of the hGH-encoding gene was codon-optimized for *E. coli* employing the ATGme online software (Daniel et al. 2015). A nucleotide sequence specifying an N-terminal 6xHis-tag and a TEV protease cleavage site were added to the gene sequence. The resulting DNA sequence including *Bcu*I/*Bam*HI restriction sites (SEQ ID NO:3) was purchased from Integrated DNA Technologies, Inc. The *Bcu*I/*Bam*HI-digested hGH DNA-fragment was cloned into the *Bcu*I/*Bam*HI-digested pHM39 plasmid to generate pHM64. In this plasmid, hGH is under the control of the promoter J23111 (SEQ ID NO:9) and RBS Bba_B0064 (SEQ ID NO:12).

To generate an empty control plasmid, a small fragment from pHM38 containing the Strep tag and the nucleotides coding for the first eight amino acids of sf-GFP were amplified from pHM38 using primers DNA_174 and DNA_53.

DNA_174 (SEQ ID NO:28):

ATAATGAATTCTATAGGTACCATAAATAAGGTTTTCCCCTGATGCTGCCACGCGT
GACTAGTATGTGGAGCCACCCGCAG

DNA 053 (SEQ ID NO:29):
TTTGGTCTCAAGCTTGGATCCAGTGAAAAGTTCTTCTCC

These primers put a scrambled DNA sequence with the same length of the J23111 promoter in front of the gene. The scrambled fragment has no promoter function and therefore serves as negative control for 'no gene expression'. The fragment was cut via *EcoRI*/*BamHI* and cloned into the similarly cut pHM38 to generate pHM42.

Proteins were extracted from the soluble and insoluble fractions of *E. coli* and *H. marinus* cell lysates from strains carrying either the pHM64 (SEQ ID NO:27) plasmid coding for hGH under control of a moderate promoter-RBS combination or an empty vector control (pHM42, SEQ ID NO:26). The extracted proteins of both *E. coli* and *H. marinus* rhGH expressing strains were separated via SDS-PAGE and subjected to Western Blot analysis. The N-terminal His-tag of rhGH was employed for immunological detection. Proteins were separated via SDS page as described above. A Low-Fluorescence PVDF Transfer Membrane (0.2 µm, Thermo Scientific^{™}) was activated in methanol for 15 sec and washed in ddH₂O for 2 min followed by an equilibration step in Towbin buffer (25 mM Tris, 192 mM Glycine and 25% (v/v) methanol) for 15 min. Proteins were transferred to the membrane via a semi-dry transfer system (Trans-Blot SD Semi-Dry Transfer Cell, Biorad) with Towbin buffer at 50 mA for 1 hr. The membrane was blocked in TBST + 5% BSA (20 mM Tris base, 150 mM NaCl, 0.1% Tween 20 (v/v), 5% BSA (w/v)) for 1 hr at 4°C. The 6x-His Tag Monoclonal Antibody (HIS.H8), HRP (Thermo Fisher Scientific) was diluted 1/1000 in TBST + 5% BSA and the membrane was probed overnight at 4°C. The membrane was washed three times in TBST for 10 minutes. 20 mL freshly prepared staining solution (200 mg/L diaminobenzidine (DAB), 100 mg/L CuSO₄, 5 mg/L NiSO₄×6H₂O, 0.6% H₂O₂) were added to the membrane and incubated at room temperature till protein bands became visible. The reaction was stopped by washing the membrane in ddH₂O.

Results: The results of the immune detection are set out in Fig. 3. The results revealed a strong band at the size corresponding to rhGH for the *E. coli* protein extract. A band at the same height was detected in the insoluble fraction of the *H. marinus* protein extract indicating that both strains produced the hormone.

### LITERATURE

1. Nishihara, H., Igarashi, Y., and Kodama, T. (1989) Archives of Microbiology 152, 39-43
2. Arai, H., and Ishii, M. (2019) Microbiol Resour Announc 8
3. Jo, B. H., Hwang, B. H., and Cha, H. J. (2014) J Biotechnol 185, 37-38
4. Toyoda, K., Ishii, M., and Arai, H. (2018) J Biosci Bioeng 126, 730-735
5. Toyoda, K., Yoshizawa, Y., Arai, H., Ishii, M., and Igarashi, Y. (2005) Microbiology 151, 3615-3625
6. Hoff, B., Plassmeier, J., Blankschien, M., Letzel, A. C., Kourtz, L., Schroder, H., Koch, W., and Zelder, O. (2021) Angew Chem Int Ed Engl 60, 2258-2278
7. Kachel, B., and Mack, M. (2020) Metab Eng
8. Nishihara, H., Igarashi, Y., and Kodama, T. (1991) Journal of fermentation and bioengineering 72, 358-361
9. Nishihara, H., Miyashita, Y., Aoyama, K., Kodama, T., Igarashi, Y., and Takamura, Y. (1997) Biochem Biophys Res Commun 232, 766-770
10. Nishihara, H., Miyata, Y., Miyashita, Y., Bernhard, M., Pohlmann, A., Friedrich, B., and Takamura, Y. (2001) Biosci Biotechnol Biochem 65, 2780-2784
11. Nishihara, H., Yaguchi, T., Chung, S. Y., Suzuki, K., Yanagi, M., Yamasato, K., Kodama, T., and Igarashi, Y. (1998) Arch Microbiol 169, 364-368
12. Kim, J. Y., Jo, B. H., and Cha, H. J. (2011) J Biotechnol 155, 312-319
13. Bacher, A., Eberhardt, S., Eisenreich, W., Fischer, M., Herz, S., Illarionov, B., Kis, K., and Richter, G. (2001) Vitam Horm 61, 1-49
14. Fischer, M., and Bacher, A. (2005) Nat Prod Rep 22, 324-350
15. Joosten, V., and van Berkel, W. J. (2007) Curr Opin Chem Biol 11, 195-202
16. Stahmann, K. P., Revuelta, J. L., and Seulberger, H. (2000) Appl Microbiol Biotechnol 53, 509-516
17. Schwechheimer, S. K., Becker, J., Peyriga, L., Portais, J. C., Sauer, D., Muller, R., Hoff, B., Haefner, S., Schroder, H., Zelder, O., and Wittmann, C. (2018) Metab Eng 47, 357-373
18. Abbas, C. A., and Sibirny, A. A. (2011) MicrobiolMolBiol Rev 75, 321-360
19. Schwechheimer, S. K., Park, E. Y., Revuelta, J. L., Becker, J., and Wittmann, C. (2016) Appl Microbiol Biotechnol 100, 2107-2119
20. Averianova, L. A., Balabanova, L. A., Son, O. M., Podvolotskaya, A. B., and Tekutyeva, L. A. (2020) Front Bioeng Biotechnol 8, 570828
21. Perkins, J., and Pero, J. (2002) Biosynthesis of riboflavin, biotin, folic acid, and cobalamin. in Bacillus subtilis and Its Closest Relatives: from Genes to Cells (Sonenshein, A., Hoch, J., and Losick, R. eds.), ASM Press, Washington DC. pp 271-286
22. Acevedo-Rocha, C. G., Gronenberg, L. S., Mack, M., Commichau, F. M., and Genee, H. J. (2018) Curr Opin Biotechnol 56, 18-29
23. Mack, M., van Loon, A. P., and Hohmann, H. P. (1998) J Bacteriol 180, 950-955
24. Winkler, W. C., Cohen-Chalamish, S., and Breaker, R. R. (2002) Proc Natl Acad Sci U S A 99, 15908-15913
25. Mironov, A. S., Gusarov, I., Rafikov, R., Lopez, L. E., Shatalin, K., Kreneva, R. A., Perumov, D. A., and Nudler, E. (2002) Cell 111, 747-756
26. Pedrolli, D. B., Kuhm, C., Sevin, D. C., Vockenhuber, M. P., Sauer, U., Suess, B., and Mack, M. (2015) Proc Natl Acad Sci USA 112, 14054-14059
27. Haase, I., Grawert, T., Illarionov, B., Bacher, A., and Fischer, M. (2014) Methods Mol Biol 1146, 15-40
28. Hemberger, S., Pedrolli, D. B., Stolz, J., Vogl, C., Lehmann, M., and Mack, M. (2011) BMC Biotechnol 11, 119
29. Schwarz, J., Konjik, V., Jankowitsch, F., Sandhoff, R., and Mack, M. (2016) Angew Chem Int Ed Engl 55, 6103-6106
30. Pedrolli, D. B., Jankowitsch, F., Schwarz, J., Langer, S., Nakanishi, S., and Mack, M. (2014) Methods MolBiol 1146, 41-63
31. Jankowitsch, F., Schwarz, J., Ruckert, C., Gust, B., Szczepanowski, R., Blom, J., Pelzer, S., Kalinowski, J., and Mack, M. (2012) J Bacteriol 194, 6818-6827
32. Haase, I., Sarge, S., Illarionov, B., Laudert, D., Hohmann, H. P., Bacher, A., and Fischer, M. (2013) Chembiochem 14, 2272-2275
33. Sarge, S., Haase, I., Illarionov, B., Laudert, D., Hohmann, H. P., Bacher, A., and Fischer, M. (2015) Chembiochem 16, 2466-2469
34. Olson, K., Fenno, J., Lin, N. et al. (1981) Nature 293, 408―411
35. Bishé, B., Arnaud, T., and Golden, J. W. (2019), Iscience 20, 216-228
36. Daniel E., Onwukwe, G. U., Wierenga, R. K., Quaggin S. E., Vainio S. J. and Krause, M. (2015), BMC Bioinformatics, 16, Article number: 303

## Claims

1. Chemolithoautotrophic bacterium of the genus *Hydrogenovibrio* which has been genetically modified to produce one or more heterologous proteins.

2. Chemolithoautotrophic bacterium of claim 1, wherein the chemolithoautotrophic bacterium is of the species *Hydrogenovibrio marinus.*

3. Chemolithoautotrophic bacterium of claim 2, wherein the chemolithoautotrophic bacterium is derived from *Hydrogenovibrio* marinus MH-110, deposited at the Japanese Cell Collection (JCM) under deposition number JCM 7688.

4. Chemolithoautotrophic bacterium of claims 1-3, wherein the chemolithoautotrophic bacterium has been genetically modified to produce a mammalian protein.

5. Chemolithoautotrophic bacterium of claim 4, wherein the mammalian protein is a therapeutically active protein.

6. Chemolithoautotrophic bacterium of claim 5, wherein the mammalian protein is human somatotropin (hGH) or a biologically active fragment thereof.

7. Chemolithoautotrophic bacterium of claim 6, wherein the human somatotropin (hGH) comprises or consists of
(a) the amino acid sequence of SEQ ID NO: 1, or
(b) an amino acid sequence having at least 80% sequence identity to the amino acid sequences of SEQ ID NO:1.

8. Chemolithoautotrophic bacterium of claims 1-6, wherein the chemolithoautotrophic bacterium comprises a plasmid.

9. Chemolithoautotrophic bacterium of claim 6, wherein the plasmid is a non-integrating plasmid.

10. Method for the production of one or more heterologous proteins in a host cell, comprising
(a) providing a chemolithoautotrophic bacterium of any of claims 1-9;
(b) culturing the host cell under conditions that allow the production of the heterologous protein;
(c) obtaining the heterologous protein.

11. Method of claim 10, wherein step (b) comprises culturing the host cell in one of the following gas mixtures
(a) H₂:CO₂:O₂:N₂ = 89:10:1:0, 88:10:2:0, 87:10:1:0, or
(b) H₂:CO₂:O₂:N₂ = 77:10:10:3, 78:10:10:2, and 79:10:10:1.

12. Method of claim 10 or 11, wherein step (c) comprises disruption of the cells.

13. Method of any of claims 10-12, wherein step (c) further comprises purification of the heterologous protein.

14. Use of the chemolithoautotrophic bacterium of any of claims 1-9 as a host cell for the production of a heterologous protein.

15. Kit for the production of a heterologous protein comprising the chemolithoautotrophic bacterium of any of claims 1-9.
